# EUROPÄISCHE PATENTSCHRIFT

(11) **EP 1 064 993 B1**
(45) Veröffentlichungstag und Bekanntmachung des Hinweises auf die Patenterteilung: **25.09.2002**
(21) Anmeldenummer: 00111337.2
(22) Anmeldetag: 26.05.2000
(51) Int. Cl.: B01J 8/22, B01J 8/18, B01J 8/00, B01J 10/00, B01D 9/00, B01D 21/24, B01D 21/28, C07C 43/13

(54) **Auftriebsfreistrahlreaktor und seine Verwendung**
Free jet-lift reactor and its use
Reacteur du type "air-lift" et son utilisation

(30) Priorität: 29.06.1999 DE 19929846
(43) Veröffentlichungstag der Anmeldung: 03.01.2001
(73) Patentinhaber: Degussa AG, 40474 Düsseldorf (DE)
(72) Erfinder: Blumberg, Wolfram, Dr., 53447 Alfter (DE); Reeken, Burkhard, 46282 Dorsten (DE); Schütte, Rüdiger, Dr., 63755 Alzenau (DE); Clauss, Gottfried, Dr., 59759 Arnsberg (DE)

(56) Entgegenhaltungen:
- EP-A- 0 455 243
- EP-A- 0 846 486
- DE-B- 1 173 435
- FR-A- 2 639 844
- US-A- 3 875 248
- US-A- 3 883 311
- US-A- 4 111 829
- US-A- 4 648 999

## Beschreibung

Die Erfindung richtet sich auf einen Auftriebsfreistrahlreaktor zur Durchführung chemischer und/oder physikalischer Stoffumwandlungsprozesse in einem flüssigen Medium. Die Erfindung betrifft ferner die Verwendung des Auftriebsfreistrahlreaktors für solche Stoffumwandlungsprozesse, in welchen ein Feststoff gebildet und dieser in Form einer Suspension mit möglichst hoher Konzentration aus dem Reaktor abgezogen wird, oder in denen ein fluidisierter Feststoff im Apparat vorhanden ist und ein Reaktionsgas zugeführt wird.

Zur Durchführung chemischer und/oder physikalischer Stoffumwandlungsprozesse in einem flüssigen Medium, in welchem ein Feststoff anwesend ist oder gebildet wird und in Form einer Suspension weiteren Prozeßstufen zugeführt wird, sind unterschiedliche Reaktoren bekannt. Bei den fraglichen Stoffumwandlungsprozessen handelt es sich beispielhaft um Kristallisationsprozesse, um Reaktionen, in deren Verlauf aus gelösten monomeren Komponenten unlösliche Polymere gebildet werden oder um Reaktionen in Gegenwart eines Suspensionskatalysators, der ständig oder periodisch teilweise ausgeschleust und nach Regenerierung wieder rezykliert werden muß.

Zur Durchführung diskontinuierlicher und kontinuierlicher Kristallisationsverfahren sind unterschiedliche Reaktoren bekannt - eine Übersicht vermittelt Ullmann's Enzyclopedia of Industrial Chemistry, 5th ed. (1988), Vol. B2, 3-22 bis 3-25. Bekannt sind beispielsweise Kristaller (siehe Fig. 24, Seite 3-22), welche einen erzwungenen externen Umlauf umfassen, wobei in einer Kreislaufleitung eine Pumpe und ein Wärmetauscher angeordnet sind und aus dem Kristaller eine Kristallsuspension abgezogen wird. Durch das mechanische Umwälzorgan kommt es leicht zu Schaumproblemen und ferner zu sekundärer Keimbildung und dadurch zu einer zu feinen Produktqualität; im Wärmetauscher besteht zudem die Gefahr einer Krustenbildung.

Zur kontinuierlichen Vakuumkristallisation ist auch der sogenannte Svenson DTB-Kristallisator (siehe loc. cit., Fig. 27, Seite 3-22) geeignet: Dieser gezeigte Vakuumkristallisator benötigt zwar nicht zwingend die dargestellte externe Zirkulationspumpe, jedoch sind ein Leitrohr sowie ein darin angeordnetes mechanisch betriebenes Umwälzorgan erforderlich. Frischlösung wird in das Leitrohr eingespeist, gebildete Kristallsuspension kann aus dem unteren Teil des Reaktors klassierend oder nichtklassierend abgezogen werden. Eine feststoffarme oder feststofffreie Lösung kann aus dem zwischen der Reaktorwandung und einem weiteren Leitblech gebildeten Ringraum über einen Überlauf abgezogen werden. Mittels dieses Kristallisatortyps ist es möglich, grobe Kristalle zu erzeugen und eine Kristallsuspension aus dem Reaktor auszuschleusen, deren Feststoffkonzentration höher ist als die Feststoffkonzentration, die sich in einem idealen, das heißt, gradientenfreien Rührkessel ergeben würde. Nachteilig an diesem Reaktor ist das Erfordernis eines mechanisch betriebenen Umwälzorgans, an welchem es leicht zu Verkrustungen sowie zu Problemen infolge unzureichender Mischung kommen kann. Im Falle einer Steigerung der Rührerdrehzahl kommt es jedoch wieder zur Kornzerstörung und gegebenenfalls zu Schaumproblemen.

Figur 28 des vorgenannten Dokuments (loc. cit., Seite 3-25) zeigt einen Vakuumkristallisator nach Standard Messo: In diesem Wirbelkristallisator sind im Reaktor zwei konzentrische Rohre angeordnet, und zwar ein inneres Leitrohr mit einem mechanisch betriebenen Umwälzorgan und ein äußeres Ejektorrohr mit einem umlaufenden Spalt, wodurch sich zwei Suspensionskreisläufe ergeben. Im inneren Kreislauf mit schneller Aufströmung im inneren Leitrohr liegt vorwiegend feineres Gut vor; diese Suspension strömt dann in dem zwischen dem inneren Leitrohr und dem Ejektorrohr gebildeten Ringraum nach unten und saugt durch den Spalt Suspension aus dem zweiten Kreislauf an. Ein Teil des Abwärtsstromes steigt im äußersten Ringraum wieder auf. Kristallsuspension mit groben Kristallen wird aus dem unteren Teil des Reaktors abgezogen, feststoffarme Lösung über einen Überlauf aus dem oberen Bereich des äußeren Ringraums des Reaktors. Die Nachteile dieses Vakuumkristallisators sind vergleichbar mit jenen des zuvor geschilderten Kristallisators, hinzukommt jedoch noch ein technisch aufwendigerer Aufbau. Verkrustungen auf den inneren Einbauten und Wänden beeinträchtigen aufgrund der relativ kleinen Spalte die Fluiddynamik, was zu einer mäßigen Standzeit des Apparates führt.

Wesentliche Probleme der zuvor gewürdigten Kristallisatoren werden durch einen Auftriebsfreistrahlreaktor, wie er aus der EP-A 0 846 486 bekannt ist, behoben. Dieser Auftriebsfreistrahlreaktor umfaßt einen von Einbauten im wesentlichen freien, sich im unteren Teil verjüngenden Behälter mit einer Vorrichtung zur Gasinjektion in einer eine Auftriebsfreistrahlumwälzung ermöglichenden Positionierung, ferner ein Einleitungsrohr zum Zuführen eines flüssigen Mediums sowie im unteren Teil des Reaktors einen Stutzen zur Abnahme einer Suspension. Da der Reaktor im wesentlichen frei von Einbauten, wie Leitblechen, Rührer und dergleichen ist, erlaubt er eine ungestörte Großraumzirkulation. Da Verkrustungen die Fluiddynamik kaum beeinträchtigen, wird eine längere Standzeit erzielt. Eine Schaumbildung, wie sie bei vielen chemischen und physikalischen Stoffumwandlungsprozessen in einem flüssigen Medium auftritt, kann durch Verwendung eines solchen Auftriebsfreistrahlreaktors vermieden oder vermindert werden. Nachteilig an diesem Reaktor ist, daß die Feststoffkonzentration der aus dem Reaktor ausgetragenen Suspension nicht höher ist als die Feststoffkonzentration eines idealen Rührkessels. Im Falle der Kühlungskristallisation von Stoffen, welche eine geringe Löslichkeitsdifferenz zwischen Ein- und Austrittszustand, zum Beispiel durch insgesamt geringe Löslichkeit aufweisen, ist somit die Feststoffkonzentration der ausgetragenen Suspension gering, und es müssen sich demgemäß gegebenenfalls aufwendige weitere Maßnahmen zur Konzentrierung der Suspension anschließen. Ähnliches gilt für feststoffbildende Reaktionen (Fällungskristallisation) mit verdünnten Edukten.

Ein weiterer Kristallisator ist aus der EP-A 0 455 243 bekannt: In dem sich nach unten verjüngenden Behälter sind eine Vorrichtung zur Gasinjektion sowie axial im Behälter ein Leitrohr angeordnet, wobei letzteres aber unterhalb der Flüssigkeitsoberfläche endet. Gesättigte Lösung wird dem Leitrohr zugeführt. Das injizierte Gas führt zu einer Aufwärtsströmung im Leitrohr; eine Abwärtsströmung der Suspension erfolgt im Ringraum außerhalb des Leitrohrs. Die gebildete Suspension wird am tiefsten Punkt des Behälters abgezogen. Die Feststoffkonzentration der abgezogenen Suspension entspricht im wesentlichen derjenigen eines idealen Rührkessels. Ebenso findet hier keine Klassierung statt, das heißt, die Korngrößenverteilung der abgezogenen Suspension ist gleich der mittleren des Behälterinhalts.

Aufgabe der vorliegenden Erfindung ist demgemäß, einen Auftriebsfreistrahlreaktor bereitzustellen, womit nicht nur die Bildung eines Feststoffs in kontrollierter Weise und ohne Zerkleinerung mittels mechanischer Mittel erfolgen kann, sondern gleichzeitig eine Konzentrierung der abgezogenen Suspension möglich ist. Weitere Aufgaben ergeben sich aus der weiteren Beschreibung der Erfindung.

Die Aufgabe wird gelöst durch einen Auftriebsfreistrahlreaktor (1) zur Durchführung chemischer und/oder physikalischer Stoffumwandlungsprozesse in einem flussigen Medium, umfassend einen von bewegten Teilen freien Behälter mit einem sich nach unten verjüngenden Unterteil (3) und einem Oberteil (2), eine im unteren Teil des Behälters angeordnete, eine Auftriebsfreistrahlumwälzung ermöglichende Vorrichtung zur Gasinjektion (6), eine Vorrichtung zum Zuführen eines flüssigen Mediums (8), dessen Auslaßöffnung (9) sich im Bereich der Kernströmung des sich im Betriebszustand ausbildenden Blasenschleiers befindet, und eine Vorrichtung (5) zur Abnahme einer Suspension am sich verjüngenden Teil des Reaktors, der dadurch gekennzeichnet ist, daß der ein zylindrisch ausgebildetes Oberteil und nur ein einziges darin axil angeordnetes Leitrohr (10), dessen Oberkante im Betriebszustand oberhalb des Flüssigkeitsspiegels und dessen Unterkante außerhalb der im Betriebszustand gebildeten Kernströmung des Blasenschleiers liegt, aufweist und eine Vorrichtung (11) zum Abziehen eines flüssigen Mediums aus dem zwischen der Wand und dem Leitrohr gebildeten Raum angeordnet ist. Die Unteransprüche richten sich auf bevorzugte Ausführungsformen des Reaktors.

Der Behälter des Reaktors ist so ausgestaltet, daß mittels einer punkt- oder linienförmigen Gasinjektion, welche zweckmäßigerweise am tiefsten Punkt des Reaktors angeordnet ist, durch den sich bildenden Auftriebsfreistrahl eine großräumige Umwälzung des Reaktorinhalts möglich ist. Durch den sich verjüngenden unteren Teil des Reaktors wird die Umwälzung begünstigt. Obgleich sich auch zylindrische Reaktoren mit einem Klöpperboden im unteren Teil verjüngen, handelt es sich bei der Verjüngung eines anspruchsgemäßen Auftriebsfreistrahlreaktors vorzugsweise um eine wesentlich deutlichere Verjüngung, insbesondere eine kegelförmige Verjüngung. Bei Verwendung eines Reaktors mit einer derartigen Verjüngung kommt es bei Suspensionen zur Ausbildung eines Suspensionstrennschnitts, d.h. zu einer Klassierung der in der abgezogenen Suspension enthaltenen Feststoffpartikel. Besonders bevorzugt umfaßt der Reaktor ein zylinderförmiges Oberteil und ein konisch ausgebildetes Unterteil, wobei gemäß einer weiter bevorzugten Ausführungsform das Unterteil mindestens die Hälfte des umzuwälzenden Flüssigkeitsvolumens aufnimmt. Der Öffnungswinkel des Konus liegt im allgemeinen im Bereich von 45 bis 150°, insbesondere 60 bis 120° und vorzugsweise 60 bis 100°. Zum Abführen einer feststoffreichen Suspension sind am konischen Unterteil ein oder mehrere Stutzen in gleicher oder gegebenenfalls unterschiedlicher Höhe angeordnet.

Der Reaktorbehälter ist frei von bewegten Teilen; in ihm sind somit keine Rührorgane enthalten. Desweiteren enthält der Reaktor auch keine Mittel zur Umwälzung des Reaktorinhalts über einen pumpenbetriebenen externen Kreislauf.

Obgleich in der EP-A 0 846 486 ausdrücklich gelehrt wird, daß der Reaktor im wesentlichen frei sein soll von Einbauten, wie Leitblechen, wurde gefunden, daß ein einziges axil im Oberteils des Reaktors angeordnetes Leitrohr die Funktion des Reaktors gegenüber dem Stand der Technik dergestalt verbessert, daß eine feststoffreichere Suspension abgezogen werden kann, wenn gleichzeitig eine feststoffarme oder feststofffreie Klarlösung aus dem zwischen der Reaktorwand und dem Leitrohr gebildeten Raum (= Beruhigungsraum) abgezogen wird. Diese Funktion ist insbesondere dann von Vorteil, wenn der Reaktor zur Kühlungskristallisation eines Produktes verwendet wird, das selbst nur eine geringe Löslichkeit aufweist, oder für eine Fällungskristallisation mit verdünnten Edukten eingesetzt wird. Nicht nur der Suspensionsaustrag sondern auch der gesamte Behälterinhalt ohne Klarzone zwischen Reaktorwand und Leitblech weist gegenüber dem sich in einem idealen Rührkessel ergebenden Feststoffgehalt einen erhöhten Feststoffgehalt auf. Dadurch ergibt sich für eine geforderte Feststoffverweilzeit ein gegenüber einem idealen Rührkessel reduziertes Apparatevolumen. Auf diese Weise sind zur Aufarbeitung der abgezogenen feststoffreicheren Suspension geringer dimensionierte und damit wirtschaftlichere Apparate erforderlich. Gleichzeitig kann die Klarlösung als Mutterlauge in den Prozeß zur Bildung des zu kristallisierenden Stoffes zurückgeführt werden.

Im Reaktor mit zylinderförmigem Oberteil ist das Leitrohr, axial und konzentrisch angeordnet.

Die Unterkante des Leitrohrs ist derart angeordnet, daß sie außerhalb der durch die Gasinjektion bewirkten Kernströmung des sich ausbildenden Blasenschleiers liegt. Vorzugsweise liegt die Unterkante des Leitrohrs im wesentlichen außerhalb des Blasenschleiers, so daß in den zwischen der Wand und dem Leitrohrs gebildeten Raum - im Falle eines zylindrischen Oberteils und einem darin parallel und axial angeordneten Leitrohr handelt es sich um einen Ringraumim wesentlichen keine oder zumindest nur in sehr geringem Umfang Blasen aufsteigen. Die Oberkante des Leitrohrs liegt oberhalb und vorzugsweise deutlich oberhalb des Flüssigkeitsniveaus im Betriebszustand. Ein Übertritt von Suspension und siedebedingten Suspensionströpfchen aus dem innerhalb des Leitrohrs gebildeten Innenraum des Reaktors in die außen liegende Beruhigungszone wird damit vermieden. Die Oberkante des Leitblechs kann auch direkt mit der Wand des Oberteils des Reaktors verbunden werden.

Die Wand des Oberteils des Reaktors enthält im Bereich des aus der Wand des Oberteils und dem Leitrohr gebildeten Raums einen oder mehrere Abzugsstutzen oder andere geeignete Mittel zum Abführen einer Klarlösung aus dem Reaktor. Ein solches Mittel ist eine an der Innenwand des Oberteils des Reaktors umlaufende Überlaufrinne mit einem Abzugsstutzen. Außer dem Leitrohr einschließlich seiner Halterungen sowie einer gegebenenfalls eingebauten Überlaufrinne für die Klarlösung und einem Mittel zum Zuführen eines flüssigen Mediums enthält der Reaktor in der Regel keine weiteren Einbauten. Im Unterteil des Reaktors, zweckmäßigerweise am tiefsten Punkt, sind jedoch eine Vorrichtung zur Gasinjektion, an der Wand des sich verjüngenden Unterteils ein oder mehrere Stutzen zur Abnahme einer feststoffreichen Suspension, an der Wand des Oberteils ein oder mehrere Stutzen zur Abnahme einer Klarlösung und im Oberteil des Reaktors außerhalb des Flüssigkeitsniveaus ein Stutzen zum Abzug der Brüden angeordnet.

Die Breite des zwischen der Wand des Oberteils des Reaktors und dem Leitrohr gebildeten Spalts liegt üblicherweise im Bereich von weniger als 25 % bis mindestens 2,5 % der Breite des Oberteils. Vorzugsweise liegt die Spaltbreite im Bereich von 5 bis 20 %, insbesondere 10 bis 20 %, des Durchmessers eines Reaktors mit zylindrischem Oberteil. Der Fachmann wird die Spaltbreite so wählen, daß die Sinkgeschwindigkeit der Partikel größer ist als die Steiggeschwindigkeit des flüssigen Mediums im Spalt.

Die Vorrichtung zum Einleiten des flüssigen Mediums ist im allgemeinen rohrförmig ausgebildet und die Auslaßöffnung befindet sich im Bereich des im Betriebszustand gebildeten Blasenschleiers, zweckmäßigerweise in der Kernströmung. Die Vorrichtung zum Zuführen eines flüssigen Mediums kann auch in die Vorrichtung zur Gasinjektion integriert werden: Beispielsweise kann das Einleitungsrohr für das flüssige Medium ein Einleitungsrohr für das zu injizierende Gas zentral durchdringen, so daß das Gas durch den gebildeten Ringspalt in den Reaktor injiziert wird. Durch unterschiedliche Höhenpositionierung der Austrittsstelle des Einleitungsrohrs für das flüssige Medium läßt sich die Lage des Suspensionstrennschnitts beeinflussen.

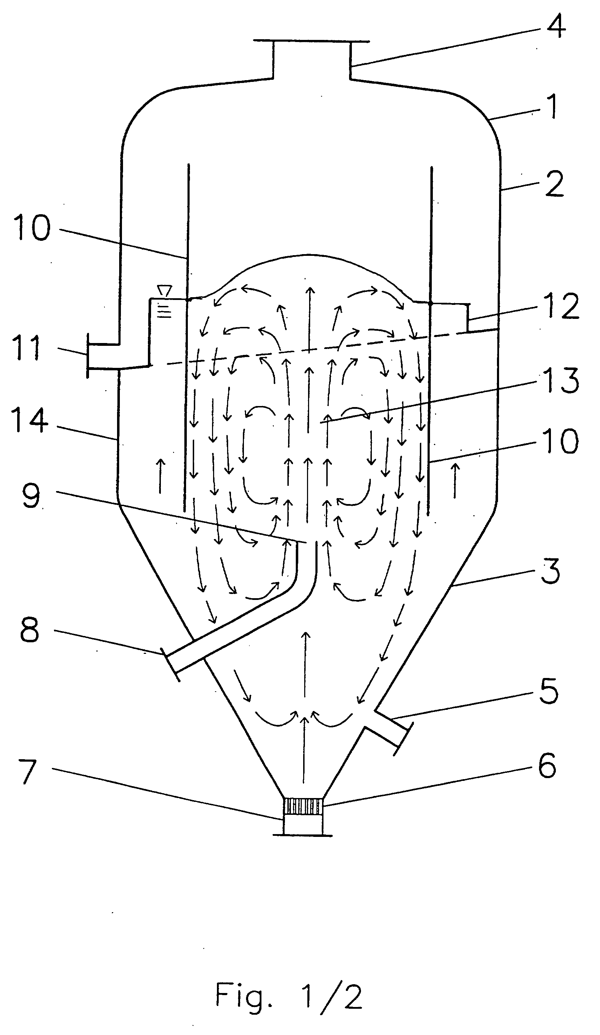

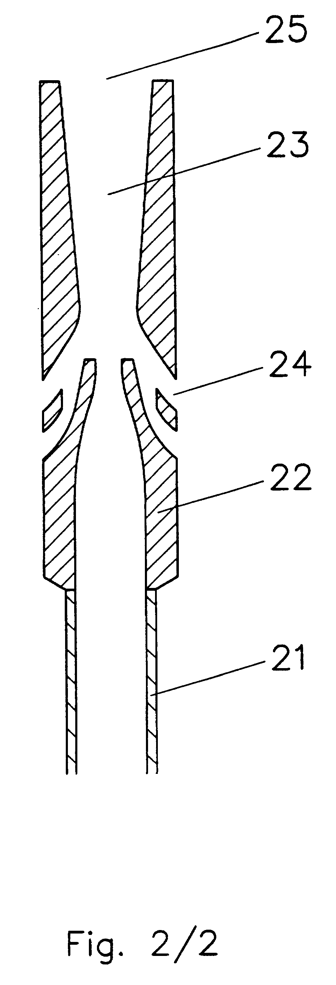

Figur 1/2 zeigt eine schematische Darstellung einer bevorzugten Ausführungsform eines erfindungsgemäßen Auftriebsfreistrahlreaktors. Der Reaktor 1 umfaßt ein zylindrisches Oberteil 2, ein konisches Unterteil 3, einen Stutzen 4 zum Abzug von Brüden, einen Stutzen 5 zum Abzug einer feststoffreichen Suspension, eine Lochplatte 6 als Gasinjektionsvorrichtung mit dem Gaszuleitungsrohr 7, ein Zuleitungsrohr für ein flüssiges Medium mit der Öffnung 9, das Leitrohr 10, dessen unteres Ende sich auf der Höhe des Übergangs vom konischen zum zylindrischen Teil des Reaktors und dessen oberes Ende sich außerhalb des Niveaus des flüssigen Mediums befindet, einen Stutzen 11 zur Abnahme der Klarlösung und eine innen angeordnete umlaufende Überlaufrinne 12, welche mit dem Stutzen 11 in Verbindung steht. Die Pfeile zeigen die Strömungsrichtung des flüssigen Mediums im Betriebszustand an, wobei die Kernströmung im achsnahen Bereich nach oben strömt; im oberen Bereich wird die Strömung umgelenkt und die Suspension strömt an der Innenseite des Leitrohrs abwärts. In dem Maße, wie Klarlösung aus dem Reaktor abgezogen wird, strömt diese in dem von der Reaktorwand und dem Leitrohr gebildeten Ringraum nach oben.

Gemäß einer bevorzugten Ausführungsform einer Vorrichtung zum Einleiten eines flüssigen Mediums umfaßt diese ein Rohr mit einem Endstück, wobei das Endstück einen zentralen Kanal mit einer Austrittsöffnung für das flüssige Medium und eine oder mehrere, zweckmäßigerweise radial angeordnete und in diesen zentralen Kanal mündende Bohrungen (= Ansaugkanäle) aufweist. In Figur 2/2, welche einen Längsschnitt durch eine derartige handelsübliche Vorrichtung zeigt, bedeuten 21 das Rohr, 22 das Endstück, 23 den zentralen Flüssigkeitskanal und 24 eine Bohrung (= Ansaugkanal), für das im Reaktor befindliche flüssige Medium. Die Bohrungen bilden zweckmäßigerweise'mit dem zentralen Kanal (in Strömungsrichtung gesehen) einen Winkel von weniger als 90°. Mittels einer derartigen Ausführungsform wird bei der Kühlungskristallisation ein Druckverlust unmittelbar vor dem Lösungseintritt erzeugt, der sicherstellt, daß vor Eintritt in den Kristaller der zur Flüssigkeitstemperatur gehörende Siededruck überschritten und so ein Sieden in der Rohrleitung verhindert wird. Zusätzlich wird eine Krustenbildung im Bereich der Austrittsstelle der Vorrichtung zum Einleiten des flüssigen Mediums vermieden.

Der erfindungsgemäße Auftriebsfreistrahlreaktor umfaßt vier hydraulisch getrennte Bereiche, welche sich günstig auf eine Vakuumkristallisation auswirken und gleichzeitig die Gewinnung einer feststoffreichen Suspension und eines feststofffreien/-armen Klarlaufs ermöglichen: (i) Auftriebsbereich mit der Kernströmung und dem größten Teil des Blasenschleiers; die Reaktions- und/oder Kristallisationswärme und/oder durch Abkühlung einer heißen Eintrittslösung freiwerdende Verdampfungsenthalpie gelangen damit rasch an die Oberfläche und werden mit der Vakuumkühlung abgeführt. Um eine Suspension mit vorzugsweise gröberen Partikeln zu gewinnen, wird das flüssige Medium vorzugsweise dicht oberhalb des sich ausbildenden Suspensionsschnitts eingeleitet. (ii) Abtriebsbereich im Bereich der Innenseite des Leitrohrs sowie in Wandnähe des sich verjüngenden Teil des Reaktors. Durch die Großraumzirkulation erfolgt eine Querstromklassierung, wobei feinere Partikel in den Blasenschleier eingezogen, größer werdende Partikel jedoch auf zunehmend längeren Bahnen in Richtung Spitze des sich verjüngenden Reaktorunterteils wandern. (iii) Trennschnitt der Suspensionsdichte im sich verjüngenden Unterteil. Der zur Konusspitze abnehmende Auftriebsstrom begrenzt die hydraulische Förderung an Partikeln, wodurch sich mit zunehmender Partikelgröße und Partikelanzahl und nicht zu hoher Rührgasmenge in der Höhe des hydraulischen Gleichgewichts ein Trennschnitt der Suspensionsdichte ausbilden kann.(iv) Beruhigungszone, in welcher die Geschwindigkeit der Aufwärtsströmung vorzugsweise geringer ist als die Sedimentationsgeschwindigkeit von in diesen Raum mitgerissenen Partikeln.

Der erfindungsgemäße Reaktor läßt sich vorzugsweise zur kontinuierlichen Durchführung eines Stoffumwandlungsprozesses in einem flüssigen Medium, wobei im Prozeß Feststoffe anwesend sind oder gebildet werden und wobei kontinuierlich oder pulsierend oder nach beendetem Prozeß eine Suspension abgezogen wird, deren Feststoffkonzentration größer ist als es bei einem idealen Rührkessel der Fall wäre. Demgemäß läßt sich der Reaktor vorzugsweise für solche Prozesse verwenden, in deren Verlauf ein Feststoff gebildet wird und die Löslichkeitsdifferenz zwischen Ein- und Austrittszustand, zum Beispiel durch geringe Löslichkeit im Eintrittszustand, niedrig ist. Ein Beispiel für einen solchen Prozeß ist ein Verfahren zur Gewinnung von Dipentaerythritol aus einer Mono- und Dipentaerythritol enthaltenden wäßrigen Lösung. Dipentaerythritol besitzt nur eine geringe Löslichkeit. Unter Verwendung eines erfindungsgemäßen Reaktors läßt sich das Verfahren dadurch verbessern, daß bereits im Reaktor die Feststoffkonzentration erhöht wird, indem gleichzeitig mit dem Abzug einer dipentaerythritolreichen Suspension eine feststofffreie oder feststoffarme Losung aus dem Reaktor abgezogen und der Prozeßstufe zur Herstellung Monound Dipentaerythritol enthaltenden Lösung zugeführt werden kann. Durch eine Abtrennung von Feinkorn mit dem Klarlauf ist eine Vergrößerung der mittleren Korngröße möglich.

Bei den Kristallisationsprozessen, die im erfindungsgemäßen Reaktor durchführbar sind, handelte es sich vorzugsweise um Vakuumkristallisationen, wobei dem Reaktor entweder eine gesättigte oder übersättigte Lösung des zu kristallisierenden Stoffes oder eine oder mehrere Lösungen, welche die Rohstoffe des zu bildenden und dann zu kristallisierenden Stoffs enthalten, zugeführt werden. Das eintretende Gas kann auf eine Temperatur leicht oberhalb der Behältertemperatur vorgewärmt werden, zum Beispiel durch Einmischen von Dampf, wodurch zum Beispiel im Falle der Kühlungs- oder Fällungskristallisation Verkrustungen im Bereich der Gaseinspeisung, bedingt durch Unterkühlung der gesättigten oder übergesättigten Lösung, vermieden werden kann.

Eine weitere Verwendung des Reaktors richtet sich auch auf Polymerisationsreaktionen, wobei dem Reaktor ein flüssiges Medium, das gelöste oder suspendierte Katalysatoren und eine oder mehrere monomere Verbindungen enthält, zugeführt und ein im Medium gebildetes unlösliches Polymeres in Form einer Suspension ausgetragen wird. Anstelle eines gelösten Monomeren kann ein solches auch mit dem den Auftriebsfreistrahl bildenden Gasstrom in das System eingeführt werden. Im Falle eines einen gelösten Katalysator enthaltenden flüssigen Mediums kann dieses nach Aufstockung mit einer oder mehreren Reaktanden wieder dem Reaktor zugeführt werden.

Weitere Verwendungen sind feststoff- oder feststofftragerkatalysierte Gas-/Flüssigkeitsreaktionen, etwa Hydrierungen, wobei das Reaktionsprodukt mit der im wesentlichen feststofffreien entgasten Flüssigkeit abgezogen werden kann. Der Feststoff kann zu Regenerationszwecken nach oder periodisch während der Umsetzung als Suspension abgezogen werden.

Die erfindungsgemäße Vorrichtung zeichnet sich durch eine einfache und damit kostengünstige Bauart aus. Der Reaktor ist frei von bewegten Teilen und auch bei den Einbauten handelt es sich um solche einfachster Bauart, so daß Verkrustungen innerhalb des Reaktors im wesentlichen vermieden werden. Durch die Positionierung eines Stutzens zur Abnahme einer Suspension ist es leicht möglich, eine feststoffreiche Suspension abzuziehen, deren Feststoffpartikel ein vorbestimmtes eng begrenztes Kornspektrum aufweisen. Durch den gleichzeitigen Abzug eines Klarlaufs kann die Feststoffkonzentration der abgezogenen Suspension signifikant erhöht werden. Der technische Aufwand zur Aufarbeitung der Suspension wird somit verringert.

## Patentansprüche

1. Auftriebsfreistrahlreaktor (1) zur Durchführung chemischer und/oder physikalischer Stoffumwandlungsprozesse in einem flüssigen Medium, umfassend einen von bewegten Teilen freien Behälter mit einem sich nach unten verjüngenden Unterteil (3) und einem Oberteil (2), eine im unteren Teil des Behälters angeordnete, eine Auftriebsfreistrahlumwälzung ermöglichende Vorrichtung zur Gasinjektion (6), eine Vorrichtung zum Zuführen eines flüssigen Mediums (8), dessen Auslaßöffnung (9) sich im Bereich der Kernströmung des sich im Betriebszustand ausbildenden Blasenschleiers befindet, und eine Vorrichtung (5) zur Abnahme einer Suspension am sich verjüngenden Teil des Reaktors,
**dadurch gekennzeichnet,**
**daß** der ein zylindrisch ausgebildetes Oberteil und nur ein einziges darin axil angeordnetes Leitrohr (10), dessen Oberkante im Betriebszustand oberhalb des Flüssigkeitsspiegels und dessen Unterkante außerhalb der im Betriebszustand gebildeten Kernströmung des Blasenschleiers liegt, aufweist und eine Vorrichtung (11) zum Abziehen eines flüssigen Mediums aus dem zwischen der Wand und dem Leitrohr gebildeten Raum angeordnet ist.

2. Reaktor nach Anspruch 1,
**dadurch gekennzeichnet,**
**daß** er ein konisch ausgebildetes Unterteil umfasst und die Vorrichtung (11) zum Abziehen des flüssigen Mediums im oberen Bereich des Ringspalts angeordnet ist.

3. Reaktor nach Anspruch 1 oder 2,
**dadurch gekennzeichnet**,

4. Reaktor nach einem der Ansprüche 1 bis 3,
**dadurch gekennzeichnet,**
**daß** die Breite des zwischen der Wand des Oberteils des Reaktors und dem Leitrohr gebildeten Spalts weniger als 25 % und mindesten 2,5 % der Breite des Oberteils beträgt.

5. Reaktor nach Anspruch 4,
**dadurch gekennzeichnet,**
**daß** die Spaltbreite 10 bis 20 % des Durchmessers des Reaktors mit zylindrischem Oberteil beträgt.

6. Reaktor nach einem der Ansprüche 1 bis 5,
**dadurch gekennzeichnet,**
**daß** das Unterteil konisch oder keilförmig ausgebildet ist und daß die Vorrichtung zur Gasinjektion im untersten Teil und mehrere Stutzen zur Abnahme der Suspension am Umfang in gleicher und/oder in unterschiedlicher Höhe des Konus angeordnet sind.

7. Reaktor nach einem der Ansprüche 1 bis 6,
**dadurch gekennzeichnet,**
**daß** die Vorrichtung zum Einleiten eines flüssigen Mediums ein mit einem Endstück (22) versehenes Rohr (21) umfaßt, wobei das Endstück einen zentralen Kanal (23) mit einer Austrittsöffnung (25) für das Medium und eine oder mehrere Bohrungen durch die Wand, welche in den zentralen Kanal (= Ansaugkanäle (24)) münden, aufweist.

8. Verwendung des Reaktors gemäß einem der Ansprüche 1 bis 7 zur kontinuierlichen Durchführung eines Stoffumwandlungsprozesses in einem flüssigen Medium, wobei ein Feststoff in Form einer Suspension, deren Feststoffkonzentration größer ist als die in einem idealen Rührkessel erzielbare Konzentration, aus dem unteren Reaktorteil und zusätzlich eine im wesentlichen feststofffreie Lösung aus dem zwischen der Wand des Oberteils und dem Leitrohr gebildeten Raum kontinuierlich oder gepulst abgezogen werden.

9. Verwendung gemäß Anspruch 8 zur Durchführung einer Kristallisation unter vermindertem Druck, insbesondere zur Kristallisation von Stoffen mit geringer Löslichkeit.

10. Verwendung gemäß Anspruch 9 zur Gewinnung einer dipentaerythritolreichen Suspension aus einer Mono- und Dipentaerythritol enthaltenden Lösung.

## Claims

1. An air lift free jet reactor (1) for performing chemical and/or physical materials conversion processes in a liquid medium, comprising a container free of moving parts with a downwardly tapering lower part (3) and an upper part (2), a gas injection device (6) arranged in the lower part of the container and enabling air lift free jet circulation, a device for supplying a liquid medium (8), the outlet opening (9) of which is located in the area of the core flow of the bubble veil forming in the operating state, and a device (5) for withdrawing a suspension at the tapering part of the reactor,
**characterised in that**
the [sic] comprises a cylindrically constructed upper part and only one guide tube (10) disposed axially therein, the upper edge of which lies in the operating state above the level of the liquid and the lower edge of which lies outside the core flow of the bubble veil formed in the operating state and a device (11) is disposed for withdrawing a liquid medium from the space formed between the wall and the guide tube.

2. A reactor according to claim 1,
**characterised in that**
it comprises a conically constructed lower part and the device (11) for withdrawing the liquid medium is arranged in the upper area of the annular gap.

3. A reactor according to claim 1 or claim 2,
**characterised in that**
the device for withdrawing the liquid medium takes the form of one or more ports arranged in the wall or an overflow gutter (12) with at least one discharge port (11).

4. A reactor according to one of claims 1 to 3,
**characterised in that**
the width of the gap formed between the wall of the upper part of the reactor and the guide tube amounts to less than 25% and at least 2.5% of the width of the upper part.

5. A reactor according to claim 4,
**characterised in that**
the gap width amounts to 10 to 20 % of the diameter of the reactor with cylindrical upper part.

6. A reactor according to one of claims 1 to 5,
**characterised in that**
the lower part is of conical or wedge-shaped construction and **in that** the gas injection device is arranged in the lowest part and a plurality of ports for withdrawal of the suspension are arranged at the circumference at the same and/or different levels on the cone.

7. A reactor according to one of claims 1 to 6,
**characterised in that**
the device for introducing a liquid medium comprises a pipe (21) provided with an end piece (22), wherein the end piece comprises a central duct (23) with an outlet opening (25) for the medium and one or more bores through the wall, which open into the central duct (= intake channels (24)).

8. Use of the reactor according to one of claims 1 to 7 for continuously performing a materials conversion process in a liquid medium, wherein there are discharged in continuous or pulsed manner a solid in the form of a suspension, the solids concentration of which is greater than the concentration achievable in an ideal stirred-tank reactor, from the lower reactor part and in addition a substantially solids-free solution from the space formed between the wall of the upper part and the guide tube.

9. Use according to claim 8 for performing crystallisation under reduced pressure, in particular for crystallisation of materials of low solubility.

10. Use according to claim 9 to obtain a dipentaerythritol-rich suspension from a solution containing mono- and dipentaerythritol.

## Revendications

1. Réacteur à pompage remontant (1) pour effectuer des opérations de conversion de matière de nature chimique et/ou physique dans un milieu fluide comprenant un réservoir ne contenant pas de pièce mobile, et ayant une partie inférieure (3) qui se rétrécit vers le bas et une partie supérieure (2), un dispositif d'injection de gaz (6) prévu dans la partie inférieure du réservoir, permettant une mise en circulation par un pompage remontant, un dispositif pour fournir un milieu fluide (8), dont l'orifice de sortie (9) se trouve au niveau de l'écoulement central du voile de bulles qui se développe pendant le fonctionnement et un dispositif (5) pour prendre la suspension dans la partie rétrécie du réacteur,
**caractérisé en ce que**
la partie supérieure cylindrique et seulement un unique tube de guidage (10), axial, placé dans cette partie, dont le bord supérieur en position de fonctionnement se trouve au-dessus du niveau de liquide et dont le bord inférieur se situe en dehors de l'écoulement central du voile de bulles formé pendant le fonctionnement et un dispositif (11) pour extraire un milieu fluide du volume formé entre la paroi et le tube de guidage.

2. Réacteur selon la revendication 1,
**caractérisé en ce qu'**
il comprend une partie inférieure de forme conique et le dispositif (11) pour extraire le milieu fluide qui se trouve dans la zone supérieure de l'intervalle annulaire.

3. Réacteur selon les revendications 1 ou 2,
**caractérisé en ce que**
le dispositif pour extraire le milieu fluide est un ou plusieurs ajutages réalisés dans la paroi ou une goulotte de trop-plein (12) avec au moins un ajutage d'extraction (11).

4. Réacteur selon l'une des revendications 1 à 3,
**caractérisé en ce que**
la largeur de l'intervalle formé entre la paroi de la partie supérieure du réacteur et le tube de guidage est inférieure à 25 % et est égale à au moins 2,5 % de la largeur de la partie supérieure.

5. Réacteur selon la revendication 4,
**caractérisé en ce que**
la largeur de l'intervalle représente entre 10 et 20 % du diamètre du réacteur avec la partie supérieure cylindrique.

6. Réacteur selon l'une des revendications 1 à 5,
**caractérisé en ce que**
la partie inférieure est de forme conique ou en coin et le dispositif d'injection du gaz est prévu dans la partie la plus inférieure et plusieurs ajutages pour extraire la suspension sont prévus à la périphérie à la même hauteur ou à des hauteurs différentes du cône.

7. Réacteur selon l'une des revendications 1 à 6,
**caractérisé en ce que**
le dispositif pour introduire un milieu liquide est un tube (21) muni d'un embout (22), l'embout ayant un canal central (23) avec un orifice de sortie (25) pour le milieu et un ou plusieurs perçages traversant la paroi et qui débouchent dans le canal central (canaux d'aspiration 24).

8. Application du réacteur selon l'une des revendications 1 à 7, pour effecteur en continu un procédé de transformation de matière dans un milieu fluide, une matière solide sous forme de suspension dont la concentration en matière solide est supérieure à la concentration que l'on peut obtenir dans une cuve à agitateur, idéale, est extraite de la partie inférieure du réacteur et en plus une solution pratiquement sans matière solide est extraite en continu ou de manière pulsée du volume formé entre la paroi de la partie supérieure et le tube de guidage.

9. Application selon la revendication 8 pour la mise en oeuvre d'une cristallisation sous pression réduite notamment pour la cristallisation de matière à faible solubilité.

10. Application selon la revendication 9 pour obtenir une suspension riche en dipentaérythritol à partir d'une solution contenant du mono et du dipentaérythritol.
